# EUROPÄISCHE PATENTSCHRIFT

(11) **EP 0 575 611 B1**
(45) Veröffentlichungstag und Bekanntmachung des Hinweises auf die Patenterteilung: **09.12.1998**
(21) Anmeldenummer: 92902727.4
(22) Anmeldetag: 13.01.1992
(51) Int. Cl.: A61F 13/15

(54) **MONATSBINDE**
SANITARY NAPKIN
SERVIETTE HYGIENIQUE

(43) Veröffentlichungstag der Anmeldung: 29.12.1993
(73) Patentinhaber: UNI-CHARM COMPANY LIMITED, Kawanoe-Shi, Ehime 799-01 (JP)
(72) Erfinder: MURAKAMI, Masaki, Kawanoe-shi, Ehime 799-01 (JP)
(74) Vertreter: Sperling, Rüdiger, Dipl.-Ing.
(86) Internationale Anmeldenummer: JP9200018
(87) Internationale Veröffentlichungsnummer: WO9313736

(56) Entgegenhaltungen:
- GB-A- 2 244 653
- JP-A-55 052 758
- JP-A-60 075 058
- JP-U-58 084 130

## Beschreibung

### Technisches Gebiet der Erfindung:

Die vorliegende Erfindung betrifft eine Damenbinde (bzw. Hygienevliese) gemäß dem Oberbegriff des Anspruchs 1. Eine solche Damenbinde ist z.B. aus GB-A-2 244 653 bekannt.

### Der Erfindung zugrundeliegender Stand der Technik:

Es sind Damenbinden bekannt, die eine flüssigkeitsdurchlässige obere Lage, eine flüssigkeitsundurchlässige unter Lage, einen zwischen diese gelegten flüssigkeitsabsorbierenden Kern und flügelähnliche Ansatzstücke umfassen, die von der oberen und unteren Lage gebildet werden und sich von den gegenüberliegenden Seitenrändern in einem in Längsrichtung mittleren Bereich des Kernes nach außen erstrecken.

Auch ist es bekannt, die Damenbinden an ihren gegenüberliegenden Seiten mit Ansatzstücken zu versehen, die jeweils eine Tasche aufweisen, die in Längsrichtung dehnbar sein kann oder nicht. Derartige Taschen werden vorgesehen, um das Austreten von Menstruationsflüssigkeit an den Seitenrändern der Binden zu verhindern.

Die Ansatzstücke der bekannten Binden haben jedoch die Funktion, die Binde am Schrittbereich der Unterhosen der Benutzerin zu fixieren, aber nicht die Funktion, das Austreten von Menstruationsflüssigkeit an den Seitenrändern der Binde zu verhindern. Andererseits haben die Ansatzstücke dieser Binden weder die Funktion, den Schrittbereich der Unterhosen der Benutzerin zu umschließen und dadurch die Binden an diesem zu befestigen, noch ist in zufriedenstellender Weise die Funktion vorhanden, daß die Ansatzstücke sich während der Benutzung der Binde aufstellen und dadurch als Tasche oder Barriere zum Zurückhalten der Menstruationsflüssigkeit wirken.

Demgemäß ist es Aufgabe der Erfindung, neuartige Damenbinden aufzuzeigen, die mit dergestalt verbesserten flügelähnlichen Ansatzstücken versehen sind, daß die vorstehend beschriebene Funktion des Verhinderns des Flüssigkeitsaustritts in zufriedenstellender und zuverlässiger Weise erfüllt wird.

### Beschreibung der Erfindung:

Die vorliegende Erfindung ist auf eine Damenbinde gerichtet, die eine flüssigkeitsdurchlässige obere Lage, eine flüssigkeitsundurchlässige untere Lage, einen zwischen diesen liegenden flüssigkeitsabsorbierenden Kern und Flügel einschließende Ansatzstücke umfaßt, die sich von den gegenüberliegenden Seitenrändern eines in Längsrichtung mittleren Bereiches des Kerns nach außen erstrecken, wobei wenigstens diese Flügel an ihren Unterseiten mit auf diesen aufgebrachten Klebstoff versehen sind und die Binde folgende Kennzeichen aufweist:

Die Ansatzstücke umfassen äußere Überstände der unteren Lage, die sich von gegenüberliegenden äußeren Seitenrändern des in Längsrichtung mittleren Bereiches des Kerns nach außen erstrecken und so jeweils die Flügel bilden, äußere Überstände von jeweiligen Flüssigkeitssperrlagen, die auf die oberen Flächen der jeweiligen äußeren Überstände der unteren Lage aufgelegt sind, um so die Flügel zu bilden, und die Innenbereiche der jeweiligen Flüssigkeitssperrlagen, die auf die obere Fläche der oberen Lage benachbart zu den äußeren Seitenrändern des Kerns gelegt sind, wobei die Flüssigkeitssperrlagen einerseits entlang ihrem äußerem Umfang mit der oberen Fläche der unteren Lage und andererseits entlang Linien, die von ihren inneren Seitenrändern im Abstand angeordnet sind, mit der oberen Lage verbunden sind, um so zwischen den Innenbereichen und der oberen Lage Taschen zu bilden.

Bei der Verwendung wird die erfindungsgemäße Damenbinde mit ihrer Rückseite nach unten gerichtet auf die Innenfläche des Schrittbereiches der Unterhose der Benutzerin aufgelegt, worauf die Flügel auf die Außenfläche des Schrittbereiches umgefaltet werden und die Binde durch den Klebstoff am Schrittbereich befestigt ist. Die inneren Bereiche stellen sich auf und die Taschen öffnen sich beim Befestigen der Binde an der Unterhose, da die inneren Bereiche der Ansatzstücke nach außen gezogen werden, wenn die Flügel auf die Außenfläche des Schrittbereiches umgefaltet werden.

Die auf der Oberfläche der Binde zu deren Seitenrändern fließende Menstruationsflüssigkeit wird zuverlässig in den jeweiligen Taschen aufgenommen. Daher besteht keine Gefahr, daß Menstruationsflüssigkeit in irgendeiner Menge an den gegenüberliegenden Seitenrändern der Binde austreten und die Unterwäsche der Benutzerin verunreinigen könnte.

### Kurze Beschreibung der Figuren:

- Fig. 1: ist eine teilweise ausgebrochene Draufsicht einer Ausführungsform der Damenbinde gemäß vorliegender Erfindung;
- Fig. 2: eine perspektivische Unteransicht der Binde;
- Fig..3: eine vergrößerte Schnittdarstellung entlang der Linie 3-3 in Fig. 1;
- Fig. 4: eine vergrößerte Schnittdarstellung einer weiteren Ausführungsform der Binde entlang derselben Linie wie in Fig. 3; und
- Fig. 5: eine vergrößerte Schnittdarstellung der in Fig. 3 gezeigten, an dem Schrittbereich der Unterhose einer Benutzerin befestigten Binde.

### Bevorzugte Ausführungsformen der Erfindung:

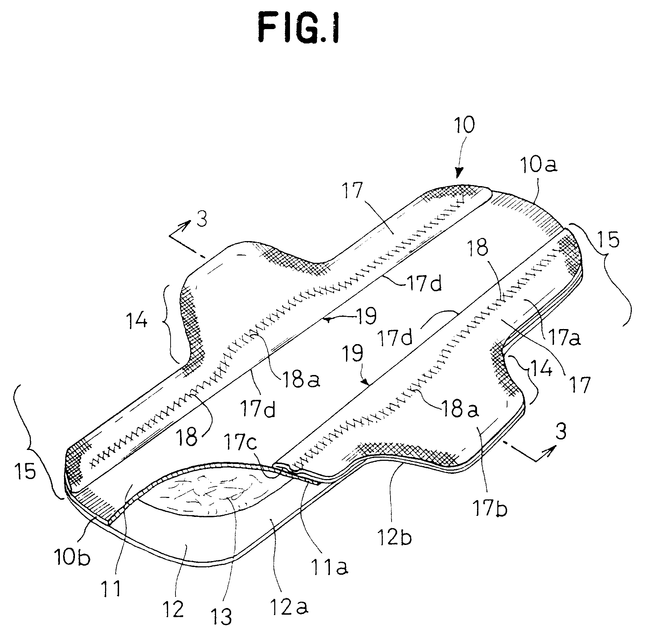

Die Erfindung ist anhand der folgenden Beschreibung bevorzugter Ausführungsformen unter Bezug auf die beiliegenden Figuren leichter zu verstehen. Eine in Fig. 1 gezeigte Binde 10 umfaßt eine flüssigkeitsdurchlässige obere Lage 11, eine flüssigkeitsundurchlässige untere Lage 12, einen zwischen diese gelegten flüssigkeitsabsorbierenden Kern 13, der sowohl in der Länge als auch in der Breite kleinere Abmessungen als die Lagen 11, 12 hat und so Überstände 11a, 12a am äußeren Umfang der jeweiligen Lagen 11, 12 bildet, und Ansatzstücke 15, die jeweils Flügel 14 einschließen.

Die untere Lage 12 ist breiter dimensioniert als die obere Lage 11, so daß die einander seitlich gegenüberliegenden Überstände 12a der unteren Lage 12 sich über die äußeren Ränder der jeweiligen seitlichen Überstände 11a der oberen Lage 11 hinaus erstrecken. Die seitlichen äußeren Überstände 11a und die zugehörigen äußeren Überstände 12a sind mittels Klebstoff (nicht dargestellt) miteinander verbunden. Die untere Lage 12 ist in ihrem in Längsrichtung gesehenen mittleren Bereich, vorzugsweise in einem geringfügig auf ein vorderes Ende 10a der Binde 10 zu versetzten Bereich, mit seitlich gegenüberliegenden zweiten äußeren Erweiterungen 12b versehen, die sich jeweils über die seitlichen äußeren Überstände 12a hinaus zur Seite erstrecken.

Die seitlich gegenüberliegenden Ansatzstücke 15, die die Flügel 14 einschließen, umfassen Flüssigkeitssperrlagen 17, die an seitlich gegenüberliegenden Seiten der Binde 10 und den äußeren Überständen 11a, 12a der oberen bzw. unteren Lage 11, 12 vorgesehen sind. Im einzelnen weisen die Flüssigkeitssperrlagen 17 Innenbereiche 17a auf, die diejenigen Bereiche der oberen Lage 11, die den gegenüberliegenden Seitenrändern des Kerns 13 benachbart sind, sowie die obere Fläche der äußeren Überstände 12a der unteren Lage 12 bedecken, äußere Überstände 17b, die die obere Fläche der zweiten äußeren Überstände 12b der unteren Lage 12 bedecken, und nach unten umgeschlagene Bereiche 17c. Die Außenränder der Flüssigkeitssperrlagen 17 sind auf die obere Fläche der unter ihnen liegenden unteren Lage 12 aufgeschweißt. Auch sind die Flüssigkeitssperrlagen 17 mittels Wärme und Druck entlang Linien, die im Abstand von durch Umschlagen nach unten gebildeten inneren Seitenrändern 17d der jeweiligen Flüssigkeitssperrlagen 17 verlaufen, mit der oberen Fläche der oberen Lage 11 in der Weise verbunden, daß diese Verbindungslinien entlang den gegenüberliegenden Seitenrändern des Kerns 13 verlaufen (Bezugszeichen 18 bezeichnet diese Linien). Auf diese Weise werden die Verbindungslinien 18 und die Taschen 19 (Fig. 5) zwischen den Innenbereichen 17a der Flüssigkeitssperrlagen 17 und der oberen Lage 11 gebildet. Die Verbindungslinien 18 sind vorzugsweise entlang den Längenabschnitten, an denen die Flügel 14 einander gegenüberliegend angeordnet sind, in Kurven nach außen gelegt, um so die jeweiligen Taschen 19 in diesen Bereichen zu vertiefen, wie durch Bezugszeichen 18a bezeichnet.

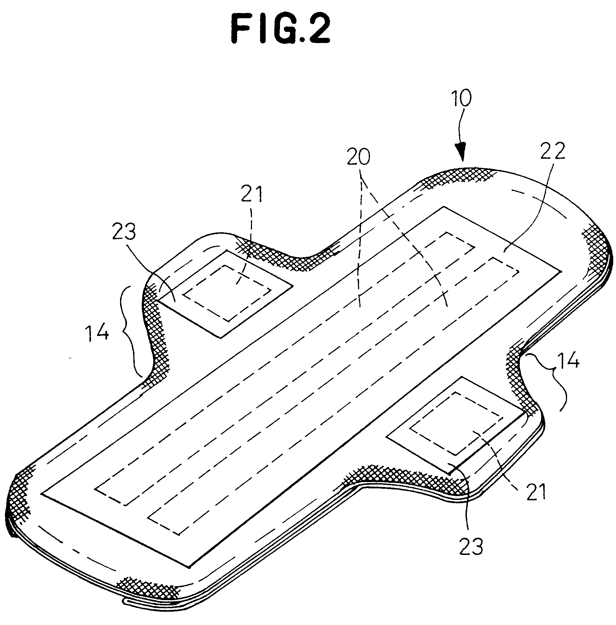

Wie Fig. 2 zeigt, ist die untere Lage 12 an der Außenfläche in ihrem Mittelbereich mit Klebstoff 20 versehen, der in zwei parallelen Streifen in Längsrichtung der unteren Lage 12 aufgebracht ist. Zusätzlich sind die jeweiligen zweiten äußeren Überstände 12b der unteren Lage 12 an ihren Außenflächen in der Mitte mit Klebstoff 21 versehen, der in Form eines Rechteckes aufgebracht ist. Diese Klebstoffstreifen bzw. -rechtecke 20 bzw. 21 sind mit abziehbaren Schutzfolien 22 bzw. 23 bedeckt.

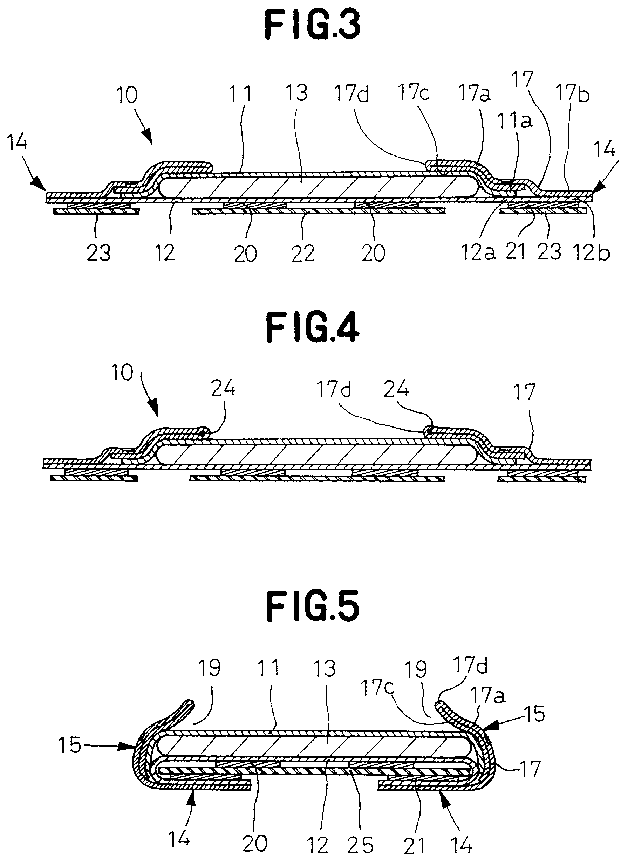

Fig. 4 zeigt eine alternative Ausführungsform, bei der die inneren Seitenränder 17d der jeweiligen Flüssigkeitssperrlagen 17 fadenartige Elemente 24 umschließen, die eine Verdickung der inneren Seitenränder 17d ermöglichen. Die fadenartigen Elemente 24 sind vorzugsweise aus weichem Gummi oder Schaumkunststoff hergestellt.

Nachfolgend wird die Verwendungsweise der Binde mit vorstehend beschriebenem Aufbau erläutert. Zunächst wird die Schutzfolie 22 abgezogen, um die Klebstoffstreifen 20 freizugeben, mit denen der Mittelbereich der Binde mit der Innenfläche eines Schrittbereiches 25 (Fig. 5) der Unterhose der Benutzerin festgeklebt wird. Anschließend werden die Schutzfolien 23 abgezogen, um die Klebstoffrechtecke 21 freizugeben und schließlich werden die jeweilige Flügel 14 umgefaltet und mittels der Klebstoffrechtecke 21 mit der Außenfläche des Schrittbereiches 25 verklebt. Bei der so an der Unterhose der Benutzerin befestigten Binde 10 werden die inneren Seitenränder 17d der jeweiligen Flüssigkeitssperrlagen 17 angehoben und die Taschen 19 sind geöffnet, wie in Fig. 5 zu sehen ist, da die Innenbereiche 17a der Flüssigkeitssperrlagen 17 mit dem Umfalten der Flügel 14 auf die Außenfläche des Schrittbereiches nach außen gezogen werden. Die auf der oberen Fläche zu den einander gegenüberliegenden Seitenrändern der Binde 10 hin fließende Menstruationsflüssigkeit wird in wirksamer Weise von den offenen Taschen 19 aufgenommen und somit besteht keine Gefahr, daß Menstruationsflüssigkeit in irgendeiner Menge durch die gegenüberliegenden Seitenränder austreten und die Unterwäsche der Benutzerin verunreinigen könnte. Die Unterflächen der jeweiligen Taschen 19, die von den äußeren Seitenrändern derselben gebildet werden, umfassen jeweils die äußeren Seitenränder der flüssigkeitsundurchlässigen unteren Lagen 12 bzw. der Flüssigkeitssperrlagen 17. Daher wird einmal in den Taschen 19 aufgenommene Menstruationsflüssigkeit in wirksamer Weise daran gehindert, durch die Unterflächen der jeweiligen Taschen 19 auszutreten.

### Gewerbliche Anwendbarkeit:

Wie aus vorstehender Beschreibung deutlich wird, kann die erfindungsgemäße Damenbinde fest an der Unterhose der Benutzerin angbracht werden, so daß sie auch bei abrupten Bewegungen der Benutzerin zuverlässig festhält. Bei der erfindungsgemäßen Binde können die Flüssigkeitssperrlagen, die die jeweils mit den Flügeln versehenen Ansatzstücke bilden, mit den gegenüberliegenden Seitenrändern der Binde selbst verbunden werden, um das Produkt fertigzustellen, so daß demgemäß eine Massenproduktion mit niedrigen Kosten ähnlich denen der herkömmlichen Binde mit flügelähnlichen Ansatzstücken möglich ist.

## Patentansprüche

1. Damenbinde (10), umfassend eine flüssigkeitsdurchlässige obere Lage (11), eine flüssigkeitsundurchlässige untere Lage (12), einen zwischen diesen liegenden flüssigkeitsabsorbierenden Kern (13) und Flügel (14) einschließende Ansatzstücke (15), die sich von den gegenüberliegenden Seitenrändern eines in Längsrichtung mittleren Bereiches des Kerns (13) nach außen erstrecken, wobei wenigstens die Flügel (14) an ihren Unterseiten mit auf diesen aufgebrachten Klebstoff (21) versehen sind,
dadurch **gekennzeichnet**, daß die Ansatzstücke (15) äußere Überstände (12b) der unteren Lage (12), die sich von gegenüberliegenden äußeren Seitenrändern des in Längsrichtung mittleren Bereiches des Kerns (13) nach außen erstrecken und so die jeweiligen Flügel (14) bilden,
äußere Überstände (12b) von jeweiligen Flüssigkeitssperrlagen (17), die auf die oberen Flächen der jeweiligen äußeren Überstände (12b) der unteren Lage (12) gelegt sind, um so die Flügel (14) zu bilden,
und die Innenbereiche (17a) der jeweiligen Flüssigkeitssperrlagen (17) umfassen, die auf die obere Fläche der oberen Lage (11) benachbart zu den äußeren Seitenrändern des Kerns (13) gelegt sind,
und daß die Flüssigkeitssperrlagen (17) einerseits entlang ihrem äußeren Umfang mit der oberen Fläche der unteren Lage (12) und andererseits entlang Linien (18), die von ihren inneren Seitenrändern im Abstand angeordnet sind, mit der oberen Lage (11) verbunden sind, um so zwischen den Innenbereichen und der oberen Lage (11) Taschen (19) zu bilden.

2. Damenbinde nach Anspruch 1, dadurch gekennzeichnet, daß die untere Lage (12) breiter dimensioniert ist als die obere Lage (11), so daß die äußeren Überstände (12a) der unteren Lage sich nach außen über die äußeren Seiten der jeweiligen äußeren Überstände (12a) der oberen Lage hinaus erstrecken.

3. Damenbinde nach Anspruch 1, dadurch gekennzeichnet, daß die äußeren Überstände (12a) der unteren Lage, die zur Begrenzung der Flügel (14) dienen, in die äußeren Seiten der jeweiligen äußeren Überstände (12a) der unteren Lage übergehen.

4. Damenbinde nach Anspruch 1, dadurch gekennzeichnet, daß die Taschen (19) so ausgebildet sind, daß sie im Bereich der Flügel (14) tiefer sind.

5. Damenbinde nach Anspruch 1, dadurch gekennzeichnet, daß die Innenbereiche der Flüssigkeitssperrlagen (17) nach unten umgeschlagen sind und durch den Umschlag geschaffene innere Seitenränder in ihnen eingeschlagene fadenartige Elemente (24) enthalten.

## Claims

1. A sanitary napkin (10) comprising a liquid-permeable topsheet (11), a liquid-impermeable backsheet (12), a liquid absorbent core (13) sandwiched therebetween, and flaps (15) including wings (14) outwardly extending from opposite side edges of a longitudinally central area of said core (13), at least said wings (14) being provided on their under sides with adhesive (21) applied thereon,
**characterized** in that said flaps (15) comprise outer extensions (12b) of said backsheet (12) extending outward from opposite outer side edges of the longitudinally central area of said core (13) so as to define said wings (14),
respectively, outer extensions (12b) of respective liquid barrier sheets (17) laid upon top surfaces of respective said outer extensions (12b) of said backsheet (12) so as to define said wings (14), and inner areas (17a) of respective said liquid barrier sheets (17) laid upon the top surface of said topsheet (11) adjacent the outer side edges of said core (13),
and that said liquid barrier sheets (17) are bonded along their outer peripheries onto the top surface of said backsheet (12) on one hand, and along lines (18) outwardly spaced from their inner side edges with said topsheet (11) so as to form pockets (19) between said inner areas and said topsheet (11), on the other hand.

2. A sanitary napkin as claimed in Claim 1, characterized in that said backsheet (12) is dimensioned wider than said topsheet (11) so that the outer extensions (12a) of said backsheet (12) extend outward beyond outer sides of the respective outer extensions (12a) of said topsheet.

3. A sanitary napkin as claimed in Claim 1, characterized in that the outer extensions (12a) of said backsheet serving to define said wings (14) are contiguous to the outer sides of the respective outer extensions (12a) of said backsheet.

4. A sanitary napkin as claimed in Claim 1, characterized in that said pockets (19) are formed so as to be deeper in the areas of said wings (14).

5. A sanitary napkin as claimed in Claim 1, characterized in that the inner areas of said liquid barrier sheets (17) are turned down and inner side edges created by such turning down contain string-like members (24) wrapped therein.

## Revendications

1. Serviette périodique (10) comprenant une couche supérieure (11) perméable aux liquides, une couche inférieure (12) imperméable aux liquides, une âme (13) absorbant les liquides, interposée entre ces couches, et des pièces ajoutées (15) englobant des ailes (14), qui s'étendent vers l'extérieur en partant des bords latéraux d'une zone médiane dans le sens de la longueur de l'âme (13), serviette dans laquelle au moins les ailes (14) sont munies, sur leurs faces inférieures, d'un adhésif (21) appliqué sur ces dernières,
**caracterisée en ce que** les pièces ajoutées (15) comprennent des projections en porte à faux (12b) extérieures de la couche inférieure (12) qui s'étendent vers l'extérieur en partant de bords latéraux extérieurs opposés de la zone médiane dans le sens de la longueur de l'âme (13), et forment ainsi les ailes (14) respectives,
des projections en porte à faux (12b) extérieures de couches de barrage aux liquides (17) respectives, qui sont posées sur les faces supérieures des projections en porte à faux extérieures (12b) de la couche inférieure (12), pour former ainsi les ailes (14)
et les zones internes (17a) des couches de barrage aux liquides (17), qui sont posées sur la surface supérieure de la couche supérieure (11), au voisinage des bords latéraux extérieurs de l'âme (13),
**et en ce que** les couches de barrage aux liquides (17) sont liées, d'une part, le long de leur pourtour extérieur, à la face supérieure de la couche inférieure (12) et, d'autre part, à la couche supérieure (11), le long de lignes (18) agencées à distance de leurs bords latéraux intérieurs, pour former ainsi des poches (19) entre les zones internes et la couche supérieure (11).

2. Serviette périodique selon la revendication 1, caractérisée en ce que la couche inférieure (12) est de dimensions plus grandes que la couche supérieure (11), si bien que les projections en porte à faux extérieures (12a) de la couche inférieure se prolongent vers l'extérieur au-delà des côtés extérieurs de chacune des projections en porte à faux extérieures (12a) de la couche supérieure.

3. Serviette périodique selon la revendication 1, caractérisée en ce que les projections en porte à faux (12a) extérieures de la couche inférieure, servant à délimiter les ailes (14), se fondent dans les côtés extérieurs de chacune des projections en porte à faux extérieures (12a) respectives de la couche inférieure.

4. Serviette périodique selon la revendication 1, caractérisée en ce que les poches (19) sont formées de façon à ce qu'elles soient plus profondes dans la zone des ailes (14).

5. Serviette périodique selon la revendication 1, caractérisée en ce que les zones internes des couches de barrage aux liquides (17) sont rabattues vers le bas et que des bords latéraux intérieurs, créés par le rabat, contiennent des éléments élastiques filamentaires (24) qui sont piqués dans ces bords.
